(19) Europäisches Patentamt
European Patent Office
Office européen des brevets

(11) Veröffentlichungsnummer: **0 008 638**
**B1**

# (12) EUROPÄISCHE PATENTSCHRIFT

(45) Veröffentlichungstag der Patentschrift: **23.09.81**

(51) Int. Cl.³: **C 07 C 97/02,** C 07 C 149/42, C 07 D 295/10, C 08 F 2/50

(21) Anmeldenummer: **79102400.3**

(22) Anmeldetag: **12.07.79**

(54) **Benzoinderivate mit quartärer Ammoniumgruppe, Verfahren zu ihrer Herstellung und ihre Verwendung.**

(30) Priorität: **15.07.78 DE 2831263**

(43) Veröffentlichungstag der Anmeldung: **19.03.80 Patentblatt 80/6**

(45) Bekanntmachung des Hinweises auf die Patenterteilung: **23.09.81 Patentblatt 81/38**

(84) Benannte Vertragsstaaten: **BE CH DE FR GB NL**

(56) Entgegenhaltungen:
**DE-B2-1 923 266**
**US-A-2 971 956**
**US-A-2 997 479**
**Chemical Abstracts, Band 62, Nr. 10, 10. Mai 1965 (COLUMBUS, OHIO, USA)**
**J. COLONGE et al. »Properties of α-ketols«**
**Spalte 116 79 B**

(73) Patentinhaber: **BASF Aktiengesellschaft, Carl-Bosch-Strasse 38, D-6700 Ludwigshafen (DE)**

(72) Erfinder: **Fischer, Martin, Dr. Chem., Elbinger Weg 1, D-6700 Ludwigshafen 29 (DE)**
Erfinder: **Kuesters, Werner, Dr. Chem., Osloer Weg 46, D-6700 Ludwigshafen (DE)**
Erfinder: **Penzel, Erich, Dr. Chem., Carl-Bosch-Strasse 86, D-6700 Ludwigshafen (DE)**
Erfinder: **Rehder, Wolfgang, Dr. Chem., Buergerm.-Horlacher-Strasse 48, D-6700 Ludwigshafen (DE)**
Erfinder: **Trautmann, Walter, Dr. Chem., Ritterbueschel 87, D-6730 Neustadt 18 (DE)**

EP 0 008 638 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Benzoinderivate mit quartärer Ammoniumgruppe, Verfahren zu ihrer Herstellung und ihre Verwendung

Die vorliegende Erfindung betrifft Benzoinderivate mit quartärer Ammoniumgruppe, ein Verfahren zur Herstellung dieser Verbindungen und ihre Verwendung als Sensibilisatoren zur Photopolymerisation polymerisierbarer Systeme, die olefinisch ungesättigte Verbindungen enthalten, insbesondere zur Herstellung wäßriger Polymerisat-Dispersionen und -Lösungen.

Durch UV-Bestrahlung polymerisierbare Gemische von olefinisch ungesättigten Verbindungen, üblichen Zusatzstoffe und Photoinitiatoren sind bekannt und finden z. B. zur Herstellung von Überzügen oder Photopolymerdruckplatten in der Praxis Verwendung. In der Patentliteratur sind eine Vielzahl von Photoinitiatoren vorgeschlagen und zum Teil auch schon praktisch eingesetzt worden, wie $\alpha$-Diketone und deren Derivate, Acyloine und deren Derivate, aromatische Disulfide oder Halogen oder Schwefel enthaltende aromatische Carbonylverbindungen.

Für die UV-initiierte Polymerisation im wäßrigen System, z. B. die photochemische Emulsionspolymerisation, bestand ein dringender Bedarf nach Photoinitiatoren mit genügender Wasserlöslichkeit. Die für den genannten Zweck bisher in der Patentliteratur beschriebenen Photoinitiatoren, z. B. Chlorophyll, Xanthophyll oder $\beta$-Carotin in Kombination mit Ascorbinsäure, Benzoin, Acetoin oder $\alpha,\alpha'$-Azodiisobutyramid haben deutliche Nachteile. So ist bei Verwendung dieser Photoinitiatoren der Polymerisationsumsatz gering bzw. die Polymerisationszeit sehr lang. Bei Verwendung von $\beta$-Carotin, Chlorophyll oder Xanthophyll in Kombination mit Ascorbinsäure treten unerwünschte Verfärbungen der Polymerisate auf.

In der DE-AS 19 23 266 werden $\alpha$-Methylolbenzoinäther beschrieben, die als Photoinitiatoren in organischen Systemen geeignet sind, jedoch für die UV-initiierte Polymerisation in wäßrigen Systemen, wie die photochemische Emulsions- oder Lösungspolymerisation infolge zu geringer Wasserlöslichkeit ungeeignet sind.

Im US-Patent 2 666 025 wird ein Photopolymerisationsverfahren beschrieben, bei dem aus einem kleinen Kreis Vinylgruppen enthaltender Monomerer Polymere mit hohen Molekulargewichten und enger Molekulargewichtsverteilung erhalten werden. Die beanspruchten Monomeren tragen am nicht endständigen $\alpha$-C-Atom der Vinylgruppe einen von Wasserstoff verschiedenen Substituenten, insbesondere Alkyl- oder Halogen-. Das Verfahren besteht darin, diese Vinylmonomeren ohne Initiator mehrere Stunden mit Licht zu bestrahlen und anschließend im Dunkeln einige Stunden oder Tage auszupolymerisieren. Photosensibilisatoren wie Bleitetraäthyl, Diacetyl, Phenylglyoxal, Glyoxal, Aceton, aliphatische Azoverbindungen werden zwar erwähnt, aber über die Wirksamkeit dieser Sensibilisatoren wird nichts ausgesagt. Das beschriebene Verfahren hat den Nachteil, daß es nur auf eine beschränkte Zahl von Monomeren anwendbar ist. Die Emulsionen sind 25%ig angesetzt. Darüber hinaus ist der Polymerisationsumsatz niedrig, wie aus den Beispielen hervorgeht: 2% Umsatz/Std. Um hohe Molekulargewichte zu erzielen, sind lange Polymerisationszeiten erforderlich. Beispielsweise muß man, um ein Molekulargewicht von $MG = 4 \cdot 10^6$ zu erzielen, 30 Stunden bei 50°C polymerisieren.

Das US-Patent 3 001 922 bezieht sich auf die Photopolymerisation von olefinisch ungesättigten Monomeren unter Verwendung von $\alpha,\alpha'$-Azodiisobutyramid als Photoinitiator. Es werden z. B. wäßrige Emulsionen mit einem Monomerengehalt von 15 Gew.-% photopolymerisiert. Nach einer 1 Stunde dauernden Bestrahlung war der Feststoffgehalt nur 0,31 Gew.-%. Bei intermittierender Bestrahlung war nach einer Stunde nur ein Feststoffgehalt von 2,1 Gew.-% erreicht.

Für die Praxis sind aber wäßrige Polymerisat-Dispersionen mit Feststoffgehalten von 30 bis 60 Gew.-%, die in vertretbaren Polymerisationszeiten erreicht werden sollen, erforderlich. Der Restmonomerengehalt soll möglichst niedrig sein. Außerdem müssen diese Dispersionen lagerstabil sein.

Der vorliegenden Erfindung lag die Aufgabe zugrunde, wasserlösliche Photoinitiatoren bereitzustellen, mit denen sich durch UV-Bestrahlung in vertretbarer Zeit und mit hohem Umsatz farblose Polymerisate herstellen lassen.

Es wurde nun gefunden, daß sich diese Aufgabe durch die erfindungsgemäßen quartären Ammoniumverbindungen lösen läßt.

Gegenstand der vorliegenden Erfindung sind quartäre Ammoniumverbindungen der Formel (I)

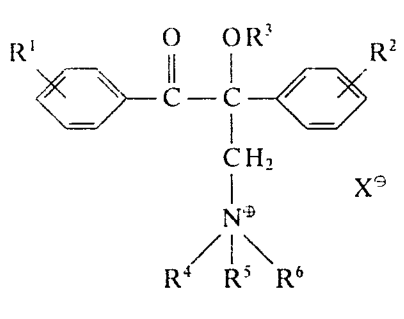

(I)

worin

$R^1$ und $R^2$ untereinander gleich oder verschieden sind und H, Halogen- oder Phenyl-, $C_1-C_6$-Alkyl-, $C_1-C_6$-Alkoxi-, oder $C_1-C_6$-Alkylthioreste bedeuten,

$R^3$ H oder einen $C_1-C_6$-Alkyl-, Hydroxyalkyl-, Cycloalkyl- oder Aralkylrest bedeutet,

$R^4$ und $R^5$ untereinander gleich oder verschieden sind und $C_1-C_6$-Alkyl-, Phenyl-, Cycloalkyl- oder Aralkylreste bedeuten oder gemeinsam eine
$-(CH_2)_4$-Brücke, eine
$-(CH_2)_5$-Brücke oder eine
$-(CH_2)_2-O-(CH_2)_2$-Brücke bilden,

$R^6$ einen $C_1-C_6$-Alkylrest und

$X^{\ominus}$ $Cl^{\ominus}$, $Br^{\ominus}$, $J^{\ominus}$, $CH_3SO_4^{\ominus}$ oder $C_2H_5SO_4^{\ominus}$

bedeuten.

Gegenstand der vorliegenden Erfindung ist außerdem ein Verfahren zur Herstellung dieser quartären Ammoniumverbindungen aus Mannichbasen der allgemeinen Formel (II)

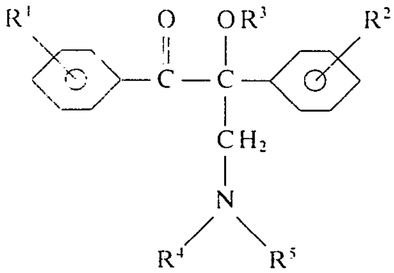

(II)

worin $R^1$ bis $R^5$ die oben angegebene Bedeutung haben, und Alkylierungsmitteln der allgemeinen Formel $R^6-X$, worin $R^6$ einen $C_1-C_6$-Alkylrest und X Cl, Br, J, $SO_4CH_3$ oder $SO_4C_2H_5$ bedeuten, in Gegenwart aprotischer Lösungsmittel.

Gegenstand der vorliegenden Erfindung ist ferner die Verwendung der erfindungsgemäßen quartären Ammoniumverbindungen als Initiatoren zur Photopolymerisation photopolymerisierbarer olefinisch ungesättigte Verbindungen enthaltender Gemische, insbesondere zur Herstellung wäßriger Polymerisat-Dispersionen oder Polymerisat-Lösungen.

Die erfindungsgemäßen quartären Ammoniumverbindungen sind neu. Sie lassen sich aus den Mannichbasen herstellen. Diese sind gemäß der Literaturvorschrift von W. Ried und G. Keil, Ann., 605, 167 f. (1957), aus Benzoinen bzw. Benzoinäthern in einer Mannich-Reaktion mi ormaldehyd und sekundären Aminen erhältlich. Einige der Mannichbasen kann man auch analog einem von G. Kinast und L. F. Tietze, Angew. Chemie, 88, 261 (1976), beschriebenen Verfahren synthetisieren (speziell 1,2-Diphenyl-2-hydroxy-3-dimethyl-amino-propan-1-on).

Diese Mannichbasen können erfindungsgemäß mit üblichen Alkylierungsmitteln wie z. B. Methylbromid oder Dimethylsulfat zu den erfindungsgemäßen Ammoniumverbindungen alkyliert werden.

Die Alkylierung wird zweckmäßigerweise in einem aprotischen Lösungsmittel bei Temperaturen von 0 bis 100°C, vorzugsweise 20 bis 70°C, durchgeführt.

In den erfindungsgemäßen quartären Ammoniumverbindungen können die Reste $R^1$ und $R^2$ an der Phenylgruppe des Benzoingerüstes in ortho-, para- oder meta-Stellung gebunden sein. Beispiele für geeignete Reste $R^1$ und $R^2$ sind außer Wasserstoff, Chlor, Brom und Phenyl, Methyl, Äthyl, Butyl, tert.-Butyl, (iso-)Propyl, Methoxyl, Äthoxyl, Methylthio, Äthylthio, Butylthio; bevorzugte Reste $R^1$ und $R^2$ sind H und Methyl.

Beispiele für den Rest $R^3$ sind außer Wasserstoff Methyl, Äthyl, Hydroxyäthyl, Hydroxypropyl, Cyclohexyl und Phenyl, vorzugsweise H, $CH_3$.

Beispiele für die Reste $R^4$ und $R^5$ sind Methyl, Äthyl, Propyl, Cyclohexyl und Benzyl, bevorzugte Reste $R^4$ und $R^5$ sind Methyl und die Brücken

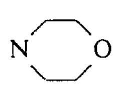

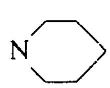

und

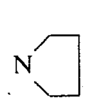

Beispiele für geeignete Reste $R^6$ sind Methyl, Äthyl, Propyl und Butyl, vorzugsweise $CH_3$. Beispiele für erfindungsgemäße quartäre Ammoniumverbindungen sind:

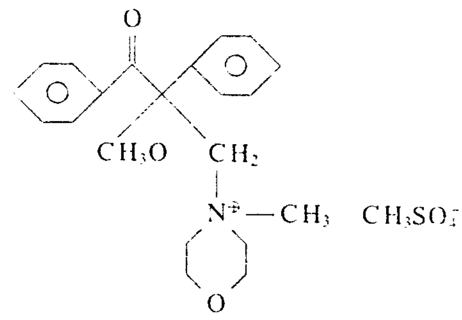

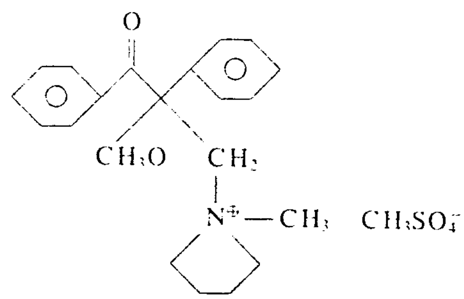

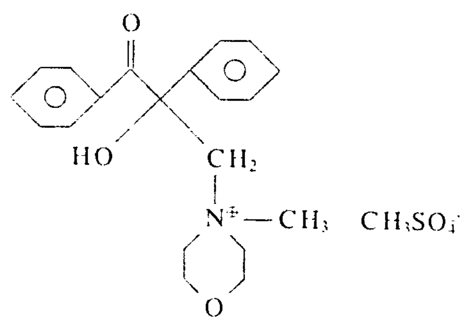

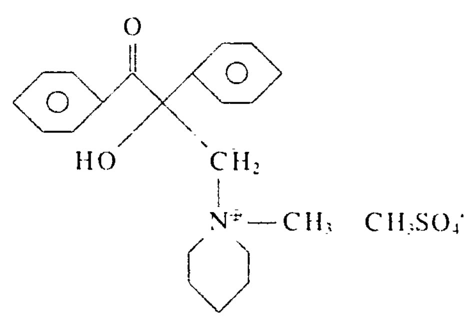

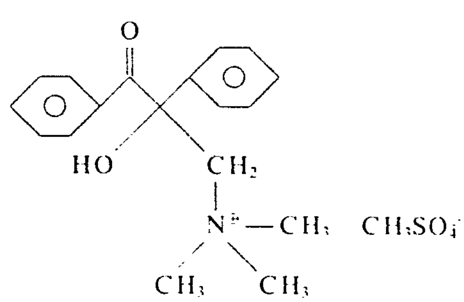

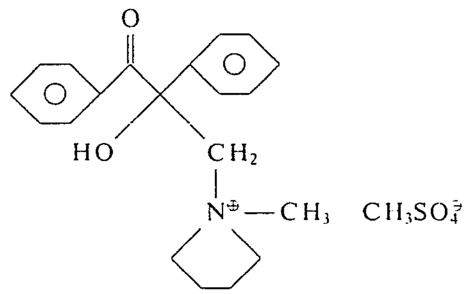

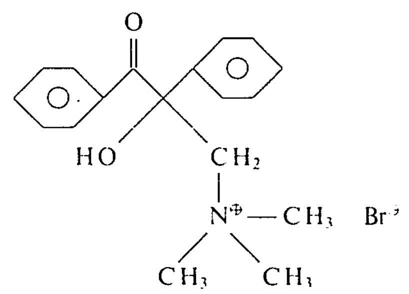

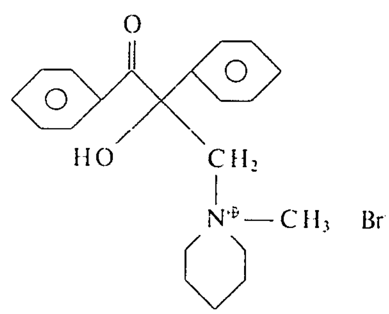

Die erfindungsgemäßen quartären Ammoniumverbindungen eignen sich als Photoinitiatoren zur Photopolymerisation polymerisierbarer, olefinisch ungesättigter Verbindungen enthaltender Gemische, insbesondere solcher, die Wasser als Lösungs- bzw. Verdünnungsmittel enthalten.

Sie eignen sich beispielsweise zur Photopolymerisation von Druckplatten auf Polyvinylalkoholbasis, vor allem zur Photopolymerisation wäßriger Emulsionen bzw. Lösungen olefinisch ungesättigter Monomerer, wobei man in vertretbarer Zeit und mit hohem Umsatz wäßrige Polymerdispersionen bzw. wäßrige Lösungspolymerisate erhält, die in der Praxis brauchbar sind.

Durch Einwirkung von UV-Licht auf wäßrige Emulsionen von olefinisch ungesättigten Monomeren in Gegenwart von Emulgatoren und/oder Schutzkolloiden und der erfindungsgemäßen quartären Ammoniumverbindungen als Photoinitiatoren lassen sich stabile wäßrige Polymerisat-Dispersionen mit hohen Feststoffgehalten herstellen.

Ebenso lassen sich aus wäßrigen Lösungen wasserlöslicher, olefinisch ungesättigter Monomerer durch Bestrahlen mit UV-Licht in Gegenwart der erfindungsgemäßen quartären Ammoniumverbindungen Lösungspolymerisate mit hohem Molekulargewicht und geringem Restmonomerengehalt herstellen.

Mit den erfindungsgemäßen Photoinitiatoren werden Umsätze von 98–100% ohne zusätzliche Peroxide oder Redoxinitiatoren erreicht. Die Lösungspolymerisate sind homogen und zeigen keine Verfärbung.

Als Lösungsmittel sind für die erfindungsgemäße Verwendung alle gängigen Lösungsmittel wie Dimethylformamid, Aceton, Tetrahydrofuran, Kohlenwasserstoffe, Alkohole, Essigsäureester usw., vorzugsweise jedoch Wasser, geeignet.

Die erfindungsgemäße Verwendung der quartären Ammoniumverbindungen zur Herstellung von Polymerisat-Dispersionen und Polymerisat-Lösungen hat auch noch folgende Vorteile: Die Polymerisation kann außer durch Kühlung zusätzlich durch Ein- und Ausschalten der Lampe gesteuert werden. Beim Ausschalten der Lampe hört die Polymerisation auf; bei erneutem Einschalten geht die Polymerisation wieder weiter. Die Polymerisationstemperatur ist in einem weiten Bereich frei wählbar. Die Polymerisationszeiten sind im allgemeinen kürzer und die nach dem erfindungsgemäßen

Verfahren hergestellten Dispersionen bzw. Lösungspolymerisate enthalten weniger Elektrolyte, die immer als Zerfallsprodukte bei den konventionellen Startersystemen anfallen.

Als olefinisch ungesättigte photopolymerisierbare Verbindungen kommen die üblichen Monomeren in Betracht, beispielsweise Mono- und Diolefine wie Äthylen, Butadien, Isopren und Chloropren, olefinisch ungesättigte Carbonsäureester wie Acryl- und Methacrylsäureester von 1 bis 12 Kohlenstoffatome enthaltenden Alkanolen oder Vinylester von 2 bis 20 Kohlenstoffatome enthaltenden Fettsäuren, Vinyl- und Vinylidenhalogenide, wie besonders Vinylchlorid und Vinylidenchlorid, vinylaromatische Verbindungen wie Styrol, $\alpha$-Methylstyrol und Vinyltoluole, Gemische derartiger Monomerer sowie Gemische solcher Monomerer mit $\alpha,\beta$-olefinisch ungesättigter Mono- und Dicarbonsäure, wie besonders Acrylsäure, Methacrylsäure, Maleinsäure, Fumarsäure und Itaconsäure, sowie deren Amide, N-Methylolamide, N-Alkoxymethylamide und Nitrile wie besonders Acrylamid, Methacrylamid, Maleinimid, Itaconsäurediamid, N-Methylolmethacrylamid, N-Methylolacrylamid, N-Methoxymethylacrylamid, N-n-Butoxymethylacrylamid, N-Methoxymethylmethacrylamid, Methylen-bis-acrylamid, Acrylnitril und Methacrylnitril. Der Anteil derartiger Comonomerer mit reaktiven Gruppen, z. B. Carboxylgruppen, in den Mischpolymerisaten beträgt im allgemeinen 0,2 bis 15, vorzugsweise 1 bis 10 Gew.-%, bezogen auf die Polymerisate. Als Comonomere kommen in untergeordneten Mengen besonders 0,2 bis 5 Gew.-%, bezogen auf die Polymerisate, auch Glykolmonoacrylat und -diacrylat, Butandiol-1,4-diacrylat, Butandiol-1,4-monoacrylat und 3-Chlor-2-hydroxypropylacrylat und -methacrylat in Betracht.

Als Beispiele für äthylenisch ungesättigte Carbonsäureester, die für die Polymerisate von besonderem Interesse sind, seien Methylacrylat, Äthylacrylat und -methacrylat, Isopropylacrylat, n-Butylacrylat, Isobutylacrylat, tert.-Butylacrylat, 2-Äthylhexylacrylat und -methacrylat, Vinylacetat, Vinylpropionat, Vinyllaurat, Vinylstearat und Vinylester verzweigter gesättigter Carbonsäuren, z. B. von Pivalinsäure, genannt.

Von besonderem Interesse sind solche wäßrigen Polymerisat-Dispersionen, die Polymerisate bzw. Mischpolymerisate enthalten, die sich von Acryl- und/oder Methacrylsäureestern 1 bis 8 Kohlenstoffatome enthaltender Alkanole und/oder von Vinylestern 2 bis 12 Kohlenstoffatome enthaltender gesättigter Monocarbonsäuren oder von Gemischen aus Butadien mit Styrol und/oder Acrylnitril ableiten und die vorzugsweise 0,5 bis 5 Gewichtsprozent, bezogen auf die Polymerisate, 3 bis 5 C-Atome aufweisende $\alpha,\beta$-olefinisch ungesättigte Carbonsäuren der obengenannten Art einpolymerisiert enthalten.

Von besonderem Interesse sind auch wäßrige Polymerlösungen, die Polymerisate bzw. Mischpolymerisate von Acrylsäure, Methacrylsäure, Acrylamid, Methacrylamid, N-Alkanolamiden der Acryl- und/oder Methacrylsäure und/oder wasserlösliche Hydroxyalkylacrylate enthalten.

Als Emulgatoren zur erfindungsgemäßen Herstellung wäßriger Polymerisatdispersionen werden im allgemeinen ionische und/oder nichtionische Emulgatoren, wie Polyglykoläther, sulfonierte Paraffinkohlenwasserstoffe, höhere Alkylsulfate wie Laurylsulfat, Alkalisalze von Fettsäuren wie Natriumstearat und Natriumoleat, Schwefelsäureester von Fettalkoholen, oxäthylierte $C_8$- bis $C_{12}$-Alkylphenole mit meist 5 bis 30 Äthylenoxidresten sowie deren Sulfonierungsprodukte sowie ferner Sulfobernsteinsäureester in Mengen, die meist zwischen 0,1 und 10,0 Gew.-%, bezogen auf die Monomeren, liegen, eingesetzt. Zusätzlich wird in manchen Fällen auch ein Schutzkolloid mitverwendet. Als Schutzkolloide kommen z. B. Polyvinylalkohol, teilverseifte Polyvinylacetate, Cellulosederivate, Copolymerisate von Acrylsäuremethylester mit Acryl- und Methacrylsäureamid oder Vinylpyrrolidonpolymerisate in Mengen zwischen etwa 0,5 und 10, insbesondere zwischen 1,0 und 5 Gew.-%, bezogen auf die Monomeren, in Frage.

Die erfindungsgemäßen Photoinitiatoren werden in Konzentrationen von 0,001 bis 10 Gew.-%, vorzugsweise 0,01 bis 1 Gew.-%, bezogen auf die olefinisch ungesättigten Verbindungen eingesetzt. In diesem Konzentrationsbereich lösen sich die Photoinitiatoren vollständig in Wasser auf.

Es können ein oder mehrere der aufgeführten Photoinitiatoren eingesetzt werden. Man kann den Photoinitiator vorlegen, aber auch in Wasser gelöst zulaufen lassen.

Als Strahlungsquelle zur Durchführung der Photopolymerisation können künstliche Strahler, deren zumindest teilweise Emission im Bereich 2000 bis 5000 Å, vorzugsweise 3000 bis 4000 Å liegt, verwendet werden. Vorteilhaft sind Quecksilberdampf-, Fluoreszenz-, Xenon- oder Wolframlampen, Leuchtstoffröhren oder Kohlenbogenlampen.

Die Durchführung der Photopolymerisation unter Verwendung der erfindungsgemäßen Photoinitiatoren kann sowohl diskontinuierlich als auch kontinuierlich geschehen. Die einzelnen Komponenten werden nach bekannten Verfahren emulgiert bzw. gelöst. Diese Emulsionen bzw. Lösungen werden dann mit Licht bestrahlt. Die Abführung der Polymerisationswärme kann durch äußere Kühlung aber auch durch Verdampfungskühlung erfolgen. Die Bestrahlungsdauer hängt von der Art der Durchführung, von der Art und Konzentration der eingesetzten Monomeren, von der Art und Menge der eingesetzten Photoinitiatoren, von der Intensität der Lichtquelle und von der Größe des Ansatzes ab und kann 5 Minuten bis 4 Stunden, vorzugsweise 10 Minuten bis 3 Stunden betragen.

Die Polymerisationstemperatur ist frei wählbar und kann +5 bis ca. 100°C betragen. Polymerisationstemperaturen von 10 bis 40°C sind besonders bevorzugt.

Die Feststoffgehalte der erfindungsgemäß hergestellten Dispersionen liegen zwischen 5 und 60

Gew.-%, im allgemeinen zwischen 30 und 50 Gew.-%. Der Gehalt an Restmonomeren ist niedrig. Die K-Werte (nach Fikentscher) der erhaltenen Dispersionen sind hoch, im allgemeinen >90. Die nach dem erfindungsgemäßen Verfahren hergestellten Dispersionen eignen sich vorzüglich zum Beschichten von Papier, Vliesstoffen und Leder. Sie sind auch geeignet für den Anstrich und als Klebemittel.

Die nach dem erfindungsgemäßen Verfahren hergestellten Lösungspolymerisate zeichnen sich durch hohe Molekulargewichte aus (K-Wert nach Fikentscher ca. 90). Sie eignen sich hervorragend als Flockungsmittel für die Abwasserreinigung, als Hilfsmittel bei der Papierherstellung und in der Textilindustrie sowie als Hilfsmittel für die tertiäre Erdölförderung.

Die in den Beispielen genannten Teile und Prozente sind, soweit nicht anders angegeben, Gewichtsteile und Gewichtsprozente.

## I. Herstellung der Mannichbasen

A: 84 Teile Benzoin
42 Teile Formalin-Lösung 40%ig
31,2 Teile Pyrrolidin

werden 10 Stunden am Rückfluß gekocht und über Nacht gerührt. Nach erneutem Aufkochen mit Äthanol wird die Lösung zur Kristallisation stehen gelassen. Der Niederschlag wird abgesaugt. Umkristallisieren aus Äthanol ergibt 57,2 Teile (49%) kristallines 1,2-Diphenyl-2-hydroxy-3-(N-pyrrolidino-)propan-1-on (NMR und Analyse bestätigen die Struktur).

B: 84 Teile Benzoin
37,5 Teile Piperidin
42 Teile Formalin-Lösung 40%ig

werden 2 Stunden am Rückfluß gekocht. Man läßt auskristallisieren und erhält nach zweimaligem Umkristallisieren aus Äthanol 91 Teile (74%) 1,2-Diphenyl-2-hydroxy-3-(N-piperidino-)propan-1-on, Fp. 69—70° C (NMR und Analyse bestätigen die Struktur).

C: 84 Teile Benzoin
40 Teile Morpholin
42 Teile Formalin-Lösung 40%ig

werden 5 1/2 Stunden am Rückfluß gekocht und über Nacht bei Raumtemperatur stehengelassen. Nach Umkristallisieren aus Äthanol erhält man 88 Teile (71,5%) 1,2-Diphenyl-2-hydroxy-3-(N-morpholino)-propan-1-on. Fp. 95—97° C (NMR und Analyse bestätigen die Struktur).

D: 22,6 Teile Benzoinmethyläther
10,5 Teile Formalin-Lösung 40%ig
9,4 Teile Piperidin

werden 6 Stunden am Rückfluß gekocht, in Chloroform aufgenommen, eingeengt und 2mal aus Methanol umkristallisiert, Ausbeute 10,1 Teile (33%) kristallines 1,2-Diphenyl-2-methoxy-3-(N-piperidino-)propan-1-on, Fp. 88° C (NMR und Analyse bestätigen die Struktur).

E: 22,6 Teile Benzoinmethyläther
10,5 Teile Formalin-Lösung 40%ig
10 Teile Morpholin

werden 6 Stunden am Rückfluß gekocht. Nach dem Abkühlen werden die ausgefallenen Kristalle mehrmals aus Äthanol umkristallisiert. Ausbeute 12,6 Teile (39%) 1,2-Diphenyl-2-methoxy-3-(N-morpholino-)propan-1-on. Fp. 95° C (NMR und Analyse bestätigen die Struktur).

## II. Herstellung der quartären Ammoniumverbindungen

### Beispiel 1

30 Teile 1,2-Diphenyl-2-hydroxy-3-(N-parrolidino)-propan-1-on, in 80 Teilen Nitromethan gelöst, werden mit 14,5 Teilen Dimethylsulfat, in 30 Teilen Nitromethan gelöst, gemischt und 2 Stunden bei 60° C gehalten. Der Niederschlag wird abgesaugt und aus Äthanol umkristallisiert. Ausbeute 18,9 Teile (45%), 1,2-Diphenyl-2-hydroxy-3-[N-(N-methyl)-pyrrolidinium]-propan-1-on-methylsulfat, Fp. 184° C (NMR und Analyse bestätigen die Struktur).

Beispiel 2

5,1 Teile 1,2-Diphenyl-2-hydroxy-3-(N-piperidino-)propan-1-on werden mit
2,1 Teilen Dimethylsulfat in
20 Teilen Dioxan vermischt.

Die Lösung wird 5 Tage bei Raumtemperatur gerührt. Die ausgefallenen Kristalle werden abgesaugt und mit Dioxan gewaschen; Ausbeute 2,3 Teile (32%) wasserlösliches kristallines 1.2-Diphenyl-2-hydroxy-3-[N-(N-methyl)-piperidinium]-propan-1-on-methylsulfat. Fp. 160–161°C (NMR und Analyse bestätigen die Struktur).

Beispiel 3

30 Teile 1,2-Diphenyl-2-hydroxy-3-(N-morpholino-)propan-1-on, in 80 Teilen Nitromethan gelöst, werden mit
14,5 Teilen Dimethylsulfat, in 30 Teilen Nitromethan gelöst, vermischt und

4½ Stunden am Rückfluß gekocht. Nach Absaugen wird der Niederschlag aus Wasser umkristallisiert; Ausbeute 18,9 Teile (45%) weiße Kristalle von 1,2-Diphenyl-2-hydroxy-3-[N-(N-methyl)-morpholinium]-propan-1-on-methylsulfat, Fp. 204°C (NMR und Analyse bestätigen die Struktur).

Beispiel 4

6,5 Teile 1,2-Diphenyl-2-methoxy-3-(N-morpholino)-propan-1-on werden mit
2,6 Teilen Dimethylsulfat in
30 Teilen Dioxan vermischt und

2 Stunden bei 60°C gerührt. Zur Vervollständigung der Umsetzung werden noch 0,7 Teile Dimethylsulfat zugegeben und 45 Minuten weitergerührt. Nach Einengen der Reaktionsmischung werden die ausgefallenen Kristalle abgesaugt und aus Cyclohexan/Äthanol umkristallisiert. Ausbeute 5 Teile (55%) 1,2-Diphenyl-2-methoxy-3-[N-(N-methyl)-morpholinium]-propan-1-on-methylsulfat. Fp. 174°C (NMR und Analyse bestätigen die Struktur).

III. Emulsionspolymerisation

Beispiel 5

Aus 50 Teilen Methylacrylat, 50 Teilen Äthylacrylat, 1,3 Teilen $C_{15}$-Paraffinsulfonat, 40%ig, 1,3 Teilen Additionsprodukt von Isooctylphenol und 25 Einheiten Äthylenoxid, 20%ig, und 210 Teilen Wasser wird in einem üblichen Mischaggregat eine Monomeren-Emulsion hergestellt.

In einem mit Thermometer und Rührer versehenen Glasgefäß befindet sich in einem Lampenschacht aus Duranglas die UV-Lichtquelle (Philips HPK 125 Watt). Zur Abführung der Polymerisationswärme wird das Reaktionsgefäß durch einen äußeren Kühlmantel mit Kühlflüssigkeit gekühlt. Der Lampenschacht wird mit Wasser gekühlt. Nach dem Einfüllen der Monomerenemulsion in das Reaktionsgefäß wird mit Stickstoff gespült. Die Temperatur der Reaktionsmischung beträgt zu Beginn 15°C. Nach Zugabe von 0,17 Teilen Photoinitiator

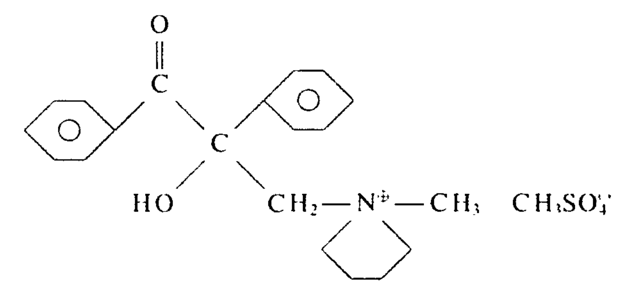

(= 0,17% bezogen auf Monomere) in 6,7 Teilen Wasser wird bestrahlt. Das Anspringen der Polymerisation macht sich durch einen Temperaturanstieg bemerkbar. Mit Hilfe der Kühlung wird die

Reaktionstemperatur zwischen 15 bis 30° C gehalten. Die Bestrahlungszeit beträgt 60 Minuten und die erhaltene wäßrige Polymerisat-Dispersion hat einen Feststoffgehalt von 30%.
Restmonomerengehalt: Methylacrylat 0,07%, Äthylacrylat 0,02%,
K-Wert des Copolymerisats: 153,6

Beispiel 6

Eine Monomerenemulsion aus 50 Teilen Methylacrylat, 50 Teilen Butylacrylat, 2 Teilen $C_{15}$-Paraffinsulfonat, 40%ig, 2 Teilen Additionsprodukt von Isooctylphenol und 25 Einheiten Äthylenoxid, 20%ig, und 89 Teilen Wasser wird mit 0,1 Teil des in Beispiel 5 genannten Photoinitiators (= 0,1% bezogen auf Monomere), gelöst in 4 Teilen $H_2O$, gemischt und wie in Beispiel 5 beschrieben photopolymerisiert. Man erhält nach 3 Stunden Bestrahlungszeit eine wäßrige Polymerisat-Dispersion mit einem Feststoffgehalt von 48,5%.
Restmonomerengehalt: Butylacrylat 0,1%, Methylacrylat 0,07%,
K-Wert des Copolymerisats: 142,2.

Beispiel 7

Eine Monomerenemulsion aus 95 Teilen n-Butylacrylat, 5 Teilen Acrylsäure, 1 Teil Alkylarylsulfonat, 15%ig, 0,33 Teilen Additionsprodukt von Isooctylphenol und 25 Einheiten Äthylenoxid, 20%ig, und 90 Teilen Wasser wird in der in Beispiel 5 angegebenen Apparatur emulgiert und nach Zugabe von 0,1 Teil des in Beispiel 5 angegebenen Photoinitiators, in 4 Teilen Wasser gelöst (0,1% Photoinitiator bezogen auf Monomere) bestahlt. Die Reaktionstemperatur liegt bei 15 bis 20°C. Die gesamte Bestrahlungsdauer beträgt 180 Minuten. Man erhält eine wäßrige Polymerisat-Dispersion mit einem Feststoffgehalt von 49%.

Restmonomerengehalt: Butylacrylat 0,1%
K-Wert des Copolymerisats: 165.

Beispiel 8

Eine Monomerenemulsion wird aus 50 Teilen n-Butylacrylat, 49 Teilen Äthylacrylat, 1 Teil N-Methylolmethacrylamid, 45%ig, 1,3 Teilen $C_{15}$-Paraffinsulfonat, 40%ig 107 Teilen Wasser in der in Beispiel 5 beschriebenen Apparatur hergestellt und durch die Emulsion Stickstoff ..eitet. Dann wird eine Lösung von 0,05 Teilen des in Beispiel 5 genannten Photoinitiators (0,05% bezogen auf Monomere), in 2 Teilen Wasser gelöst, zugesetzt und mit UV-Licht bestrahlt. Die Polymerisationszeit beträgt 135 Minuten. Die Polymerisatdispersion hat einen Feststoffgehalt von 46%.

Restmonomerengehalt: Butylacrylat 0,1%, Äthylacrylat 0,12%.

Beispiel 9 mit Vergleichsbeispielen

Eine Monomerenemulsion wird aus 75 Teilen Butylacrylat, 17 Teilen Methylmethacrylat, 4 Teilen Acrylsäure, 4 Teilen sulfoniertes Additionsprodukt von Isooctylphenol und 25 Einheiten Äthylenoxid, 35%ig in $H_2O$ gelöst, und 149 Teilen Wasser hergestellt.

In der Monomerenemulsion werden jeweils 0,23 Teile (0,24% bezogen auf Monomere) unterschiedlicher Initiatoren dispergiert und wie in Beispiel 5 beschrieben, bestrahlt. Durch Bestimmung des Feststoffgehaltes wird der Reaktionsfortgang kontrolliert. Nach Beendigung gießt man die Reaktionsmischung durch ein grobes Maschenfilter, um von entstandenem Koagulat zu trennen. Die Eigenschaften der so erhaltenen Polymerisat-Dispersionen sind in der Tabelle aufgeführt.

Im Gegensatz zu dem erfindungsgemäßen Initiator I erhält man mit anderen, dem Stand der Technik entsprechenden Initiatoren keine stabilen Dispersionen und erreicht auch nicht die hohen K-Werte.

| Initiator | Reaktionsdauer (Belichtungszeit) | Feststoffgehalt | | Koagulat-Menge | | K-Wert |
|---|---|---|---|---|---|---|
| | | theor. | gefun. | sofort | nach 3 Mon. | |
| Benzildimethylketal | 3,5 Stunden | 39% | 38% | 12 g | Dispersion setzt sich ab**) | 92 |
| Benzoinisopropyläther | 7 Stunden*) | 29% | 36% | 18 g | Dispersion setzt sich ab**) | 112 |
| β-(Benzoinäthyläther-)propionsäure | 3 Stunden | 39% | 36,5% | 10 g | Dispersion setzt sich ab**) | 130 |
| Initiator I (erfindungsgemäß) | 3 Stunden | 39% | 39% | 1 g | – | 140 |

Initiator I = 1,2-Diphenyl-2-hydroxy-3-[N-(N-methyl)-pyrrolidinium]-propan-1-on-methylsulfat.
*) Es bildet sich starker Belag am Lampenschacht.
**) Es bildet sich ein koagulierter Bodensatz.

IV. Lösungspolymerisation

Beispiel 10

Eine Lösung aus 70 Teilen Acrylsäure, 130 Teilen Acrylamid, 1800 Teilen Wasser und 1 Teil des in Beispiel 5 genannten Photoinitiators (0,5% bezogen auf Monomere) wird in der in Beispiel 5 näher beschriebenen Apparatur bei 25 bis 30°C mit UV-Licht polymerisiert. Nach 60 Minuten Bestrahlungszeit entsteht eine hochviskose, farblose Polymerisat-Lösung.

Feststoffgehalt: 10%
K-Wert: 92

**Patentansprüche**

1. Quartäre Ammoniumverbindungen der Formel (I)

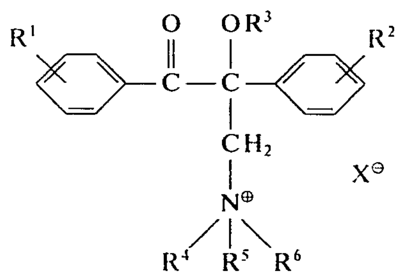

(I)

worin

| | |
|---|---|
| $R^1$ und $R^2$ | untereinander gleich oder verschieden sind und H, Halogen oder Phenyl-, $C_1-C_6$-Alkyl-, $C_1-C_6$-Alkoxi-, oder $C_1-C_6$-Alkylthioreste bedeuten, |
| $R^3$ | H oder einen $C_1-C_6$-Alkyl-, Hydroxyalkyl-, Cycloalkyl- oder Aralkylrest bedeutet, |
| $R^4$ und $R^5$ | untereinander gleich oder verschieden sind und $C_1-C_6$-Alkyl-, Phenyl-, Cycloalkyl- oder Aralkylreste bedeuten oder gemeinsam eine $-(CH_2)_4$-Brücke, eine $-(CH_2)_5$-Brücke oder eine $-(CH_2)_2-O-(CH_2)_2$-Brücke bilden, |
| $R^6$ | einen $C_1-C_6$-Alkylrest und |
| X | Cl , Br , J , $CH_3SO_4$ oder $C_2H_5SO_4$ |

bedeuten.

2. Verfahren zur Herstellung der quartären Ammoniumverbindungen nach Anspruch 1, dadurch gekennzeichnet, daß Mannichbasen der Formel (II)

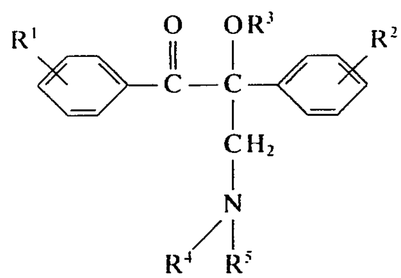

(II)

worin

| | |
|---|---|
| $R^1$ und $R^2$ | untereinander gleich oder verschieden sind und H, Halogen oder Phenyl-, $C_1-C_6$-Alkyl-, $C_1-C_6$-Alkoxi- oder $C_1-C_6$-Alkylthioreste bedeuten, |
| $R^3$ | H oder einen $C_1-C_6$-Alkyl-, Hydroxyalkyl-, Cycloalkyl- oder Aralkylrest bedeutet, |
| $R^4$ und $R^5$ | untereinander gleich oder verschieden sind und $C_1-C_6$-Alkyl-, Phenyl-, Cycloalkyl- oder Aralkylreste bedeuten oder gemeinsam eine $-(CH_2)_4$-Brücke, eine $-(CH_2)_5$-Brücke oder eine $-(CH_2)_2-O-(CH_2)_2$-Brücke bilden, |

in einem aprotischen Lösungsmittel mit Alkylierungsmitteln der allgemeinen Formel $R^6-X$, worin $R^6$ einen $C_1-C_6$-Alkylrest und X, Cl, Br, J, $SO_4CH_3$ oder $SO_4C_2H_5$ bedeuten, zu den quartären Ammoniumverbindungen umgesetzt werden.

3. Verwendung der Ammoniumverbindungen nach Anspruch 1 als Initiatoren zur Photopolymerisation polymerisierbarer olefinisch ungesättigte Verbindungen enthaltender Gemische.

4. Verwendung der Ammoniumverbindungen nach Anspruch 3 als Initiatoren zur Photopolymerisation photopolymerisierbarer Gemische, die Wasser enthalten.

5. Verwendung nach Anspruch 4 zur Herstellung wäßriger Polymerisat-Dispersionen oder Polymerisat-Lösungen.

6. Verwendung nach Anspruch 3, 4 oder 5, wobei 0,001 bis 10, vorzugsweise 0,01 bis 1 Gewichtsprozent der quartären Ammoniumverbindung, bezogen auf die olefinisch ungesättigten Verbindungen, eingesetzt werden.

7. Verwendung nach Anspruch 3, 4, 5 oder 6, wobei als Strahlungsquellen für die Photopolymerisation solche mit zumindest teilweiser Emission im Berreich von 2000 bis 5000, vorzugsweise 3000 bis 4000 Å eingesetzt werden.

## Claims

1. A quaternary ammonium compound of the formula (I)

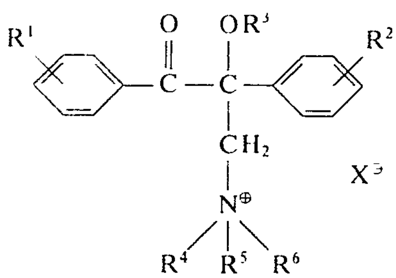

(I)

where

| | |
|---|---|
| $R^1$ and $R^2$ | are identical or different and each is H, halogen, phenyl, $C_1-C_6$-alkyl, $C_1-C_6$-alkoxy or $C_1-C_6$-alkylthio, |
| $R^3$ | is H, $C_1-C_6$-alkyl, hydroxyalkyl, cycloalkyl or aralkyl, |
| $R^4$ and $R^5$ | are identical or different and each is $C_1-C_6$-alkyl, phenyl, cycloalkyl or aralkyl, or |
| $R^4$ and $R^5$ | together are a $-(CH_2)_4$-bridge, a $-(CH_2)_5$-bridge or a $-(CH_2)_2-O-(CH_2)_2$-bridge, |
| $R^6$ | is $C_1-C_6$-alkyl, and |
| X | is Cl , Br , I , $CH_3SO_4$ or $C_2H_5SO_4$ . |

2. A process for the preparation of a quaternary ammonium compound as claimed in claim 1, characterized in that a Mannich base of the formula (II)

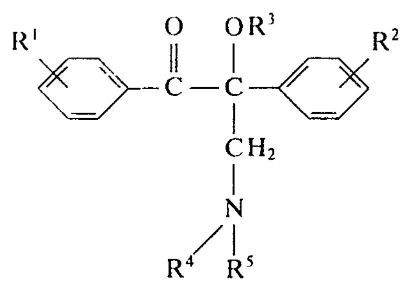

(II)

where

| | |
|---|---|
| $R^1$ and $R^2$ | are identical or different and each is H, halogen, phenyl, $C_1-C_6$-alkyl, $C_1-C_6$-alkoxy or $C_1-C_6$-alkylthio, |
| $R^3$ | is H or $C_1-C_6$-alkyl, hydroxyalkyl, cycloalkyl or aralkyl, |

| | |
|---|---|
| $R^4$ and $R^5$ | are identical or different and each is $C_1-C_6$-alkyl, phenyl, cycloalkyl or aralkyl, or |
| $R^4$ and $R^5$ | together are a $-(CH_2)_4$-bridge, a $-(CH_2)_5$-bridge or a $-(CH_2)_2-O-(CH_2)_2$-bridge, |

is reacted, in an aprotic solvent, with an alkylating agent of the general formula $R^6-X$, where $R^6$ is $C_1-C_6$-alkyl and X is Cl, Br, I, $SO_4CH_3$ or $SO_4C_2H_5$ to give the quaternary ammonium compounds.

3. Use of an ammoniumcompound as claimed in claim 1 as an initiator for the photopolymerization of a polymerizable mixture containing olefinically unsaturated compounds.

4. Use of an ammonium compound as claimed in claim 3 as an initiator for the photopolymerization of photopolymerizable mixtures containing water.

5. Use as claimed in claim 4 for the preparation of aqueous polymer dispersions or polymer solutions.

6. Use as claimed in claim 3, 4 or 5, from 0.001 to 10, preferably from 0.01 to 1, percent by weight, based on the olefinically unsaturated compounds, of the quaternary ammonium compound being employed.

7. Use as claimed in claim 3, 4, 5 or 6, there being used as radiation source for the photopolymerization one which emits at least partially in the region from 2,000 to 5,000 Å, preferably from 3,000 to 4,000 Å.

**Revendications**

1. Composés d'ammonium quaternaires de formule (I)

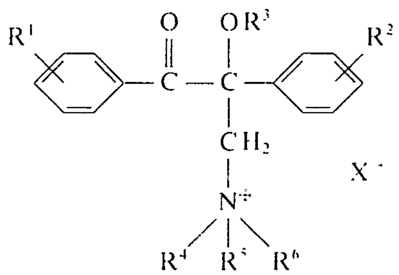

(I)

dans laquelle

| | |
|---|---|
| $R^1$ et $R^2$ | sont identiques ou différents et représentent H, halogène ou des restes phényle, alkyle en $C_1$ à $C_6$, alcoxy en $C_1$ à $C_6$, ou alkylthio en $C_1$ à $C_6$, |
| $R^3$ | représente H ou un reste alkyle en $C_1$ à $C_6$, hydroxyalkyle, cycloalkyle, ou aralkyle, |
| $R^4$ et $R^5$ | sont identiques ou différents et représentent un reste alkyle en $C_1$ à $C_6$, phényle, cycloalkyle ou aralkyle ou bien forment ensemble un pont $-(C_2)_4$, un pont $-(CH_2)_5$, ou un pont $-(CH_2)_2-O-(CH_2)_2$, |
| $R^6$ | représente un reste alkyle en $C_1$ à $C_6$ et |
| X | Cl , Br , I , $CH_3SO_4$ ou $C_2H_5SO_4$ . |

2. Procédé de préparation des composés d'ammonium quaternaires selon la revendication 1, caractérisé par le fait qu'on transforme en les composés d'ammonium quaternaires des bases de Mannich de formule (II)

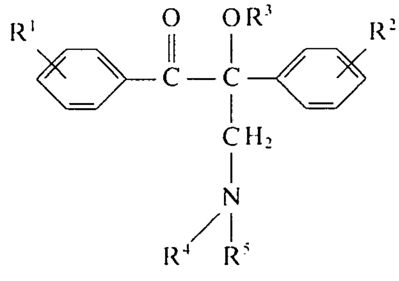

(II)

dans laquelle

| | |
|---|---|
| $R^1$ et $R^2$ | sont identiques ou différents et représentent H, halogène ou des restes phényle, alkyle en $C_1$ à $C_6$, alcoxy en $C_1$ à $C_6$, ou alkylthio en $C_1$ à $C_6$, |
| $R^3$ | représente H ou un reste alkyle en $C_1$ à $C_6$, hydroxyalkyle, cycloalkyle, ou aralkyle, |
| $R^4$ et $R^5$ | sont identiques ou différents et représentent un reste alkyle en $C_1$ à $C_6$, phényle, cycloalkyle ou aralkyle ou bien forment ensemble un pont<br>$-(CH_2)_4$, un pont<br>$-(CH_2)_5$, ou un pont<br>$-(CH_2)_2-O-(CH_2)_2$, |

dans un solvant aprotique, avec des agents d'alkylation de formule générale $R^6-X$, dans laquelle $R^6$ est un reste alkyle en $C_1$ à $C_6$ et X représente Cl, Br, I, $SO_4CH_3$ ou $SO_4C_2H_5$.

3. Utilisation des composés d'ammonium selon la revendication 1 comme initiateurs pour la photopolymérisation de mélanges contenant des composés à insaturation oléfiniques polymérisables.

4. Utilisation des composés d'ammonium selon la revendication 3 comme initiateurs de polymérisation de mélanges photopolymérisables contenant de l'eau.

5. Utilisation selon la revendication 4 pour la préparation de dispersions ou de solutions de polymérisats aqueuses.

6. Utilisation selon l'une des revendications 3, 4 ou 5, avec 0,001 à 10, de préférence 0,01 à 1% en poids de composé d'ammonium quaternaire, rapporté aux composés à insaturation oléfinique.

7. Utilisation selon l'une des revendications 3, 4, 5 ou 6, avec emploi, comme sources de rayonnement pour la photopolymérisation, de sources à émission au moins partielle dans la zone de 2000 à 5000, de préférence 3000 à 4000 Å.